# EUROPEAN PATENT SPECIFICATION

(11) **EP 1 693 034 B1**
(45) Date of publication and mention of the grant of the patent: **20.01.2021**
(21) Application number: 04819863.4
(22) Date of filing: 01.12.2004
(51) Int. Cl.: A61F 13/15

(54) **ABSORPTIVE ARTICLE**
SAUGFÄHIGER ARTIKEL
ARTICLE ABSORBANT

(30) Priority: 02.12.2003 JP 2003403180
(43) Date of publication of application: 23.08.2006
(73) Proprietor: DAIO PAPER CORPORATION, Shikokuchuo-shi, Ehime 799-0492 (JP)
(72) Inventor: MIYAIRI, Makiko c/o Elleair Paper Tech. Co. Ltd., machi Shioya-gun Tochigi 329-1411 (JP)
(74) Representative: Potter Clarkson
(86) International application number: PCT/JP2004/017872
(87) International publication number: WO 2005/053590

(56) References cited:
- EP-A1- 1 138 293
- EP-A2- 0 818 569
- WO-A1-01/49230
- WO-A1-02/03900
- WO-A1-02/051644
- WO-A1-2004/006818
- FR-A1- 2 559 037
- FR-A1- 2 733 146
- JP-A- 6 280 171
- JP-A- 2000 510 377
- US-A1- 2005 096 616

## Description

### Field of the Invention

The present invention relates to an absorbent article.

### Description of the Background

Among conventional sanitary napkins as absorbent articles, for example, a sanitary napkin including a top sheet positioned in a face which is brought into contact with a human body, a back sheet positioned in a face which is opposite to the top sheet and brought into contact with underwear, and an absorbent body interposed between the both sheets has been known.

Such sanitary napkins are designed to provide a "healing" effect or "security", to brighten up women's mood which tends to be depressed during menstruation. In a known example thereof, release paper is patterned, and a transparent wrapping material is used so that the pattern on the release paper can be seen from the outside when the napkins are individually packaged. The appearance thereof is designed to be colorful to visually distract women's mood when the napkin is being carried or used (for example, Patent Reference 1).

Patent Reference 1: Japanese Patent Laid-open publication No. 2003-199786

WO 2004/006818 A1 describes an absorbent article, which may be a sanitary napkin or a panty liner, comprising: a body contacting layer; a garment contacting layer; and an absorbent core disposed between the body contacting layer and the garment contacting layer. The absorbent core has a core edge. The core edge defines a core region within the core edge and an outer region outside the core region. The body contacting layer and the garment contacting layer extend outward into the outer region and are joined together in the outer region. In one aspect, the garment contacting layer has a graphic printed on the body facing surface at least in a portion of the outer region. In another aspect, the body contacting layer has a graphic printed on the garment facing surface at least in a portion of the outer region. The body contacting layer has a first light transmittance so that the graphic can be seen through the body contacting layer in the outer region. Since users (or women) can see the graphic through the body contacting layer, the absorbent article can provide an emotional benefit to women before uses, and thus decrease women's melancholic mood.

WO 01/49230 A1 describes an absorbent article which includes an outer cover graphic that appears brighter and more noticeable than present outer cover graphics. The outer cover includes a liquid impermeable inner layer and a fibrous outer layer which layers jointly define a graphic region where the inner and outer layers are intimately bonded together. The fibrous outer layer has a Light Transmittance Value in the graphic region of about 80 percent or higher, and an outer cover graphic is disposed on the inner layer in a location corresponding to the graphic region.

EP 1138293 A1 describes an absorbent article, such as a panty liner or sanitary napkin, which has at least one region that is transparent to visible light. In one embodiment to whole article is transparent.

FR 2559037 A1 describes nappy-pants for babies, consisting of an assembly formed from a layer of absorbent plastic material 2 and from a sheet of plastic material 1 acting as impermeable pants, characterised in that the sheet of plastic material 1 is transparent or translucent and in that it comprises visual means 6 which can be obliterated upon contact with water or urine and which, when they are obliterated 9, give an indication, from the outside, that the baby is wet, without it being necessary to undo the nappy.

FR 2733146 A1 describes a saturation indicator for an absorbent article, such as a baby's nappy. The indicator (6) contacts the absorbent layer (4) of the nappy and comprises a perforated plastic sleeve containing a strip of material which changes colour when wet. The outer impermeable layer (2) of the nappy is transparent or translucent, so that the colour of the indicator strip can be seen through it, showing when the baby needs changing. The perforated sleeve can be made as an integral part of the outer layer of the nappy, or it can be stuck to its inner layer by adhesive. The colour-change material can be made from blotting paper.

### Disclosure of the Invention

The present invention comprises an absorbent article as defined in the claims.

Problem to be Solved by the Invention

However, in the case of the disclosure of the Patent Reference 1, only symbols provided in the back sheet, or symbols dotted in the back sheet make appeal. Therefore, it could hardly be said that the visual effect thereof was adequate. To more visually make appeal and provide "healing" or "security" to women during menstruation, it is desirable that the napkin is structured to provide more visual effects by coloring larger portion of a surface of the back sheet to provide a sense of integration with underwear, providing patterns in addition to coloring, or the like.

If the back sheet is colored in order to obtain more visual effects, the light transmittance of the back sheet is reduced. Table 1 shows measurement results of the light transmittance of colored films (hereinafter, referred to as printing films) which are used for the back sheet, such as water-tight sheet materials of polyethylene, polypropylene, and the like, liquid-impermeable non-woven sheets, and microporous sheet materials.

**Table 1**

| Printing film | Light Transmittance (%) | | |
|---|---|---|---|
| | 1 | 2 | 3 |
| Yellow Film | 31.27 | 31.33 | 31.74 |
| Blue Film | 8.96 | 8.26 | 8.55 |
| Green Film | 15.52 | 15.73 | 15.42 |
| Red Film | 10.79 | 11.11 | 10.89 |
| White Film | 34.39 | 35.05 | 35.24 |

As shown in Table, the light transmittances of the blue and red printing films are especially low and less than 15%.

On the other hand, in a manufacturing process of sanitary napkins, an optical sensor is used for inspection for the purpose of checking products. Specifically, in order to confirm that the absorbent body is surely included at a proper position, to confirm that no foreign substance is mixed, and the like, the optical sensor measures a specific area of a sanitary napkin as a final product. When the light transmittance of the area is lower than a certain value, it is concluded that misalignment of the included absorbent body, contamination of a foreign substance, or the like has occurred, and the sanitary napkin is rejected from the line. Unacceptable products among final products are thus eliminated. Specifically, for example, a product with a light transmittance of less than 15% is recognized as an unacceptable product.

Accordingly, when an air-permeable film as the back sheet is colored (especially, when it is colored blue or red), the light transmittance of the back sheet becomes less than 15%, and the product with such back sheet can be recognized as an unacceptable product when being checked by the optical sensor in the inspection process of the manufacturing process.

In other words, using the colored printing film for the back sheet in order to provide more visual effects to women has a negative effect that proper judgment of acceptable products and unacceptable products cannot be performed in the inspection process of the manufacturing process.

An object of the present disclosure is to provide an absorbent article which visually gives "healing" and "security" to women during menstruation and is properly checked by an optical sensor in an inspection process of a manufacturing process.

### Means to Solve the Problem

There is described herein, an absorbent article, including: a top sheet positioned in a face which is brought into contact with a human body; a back sheet positioned in a face which is opposite to the top sheet and is brought into contact with underwear; and an absorbent body interposed between the top sheet and the back sheet. The back sheet includes a colored area, and the colored area has a light transmittance allowing light outputted from an optical sensor to be transmitted therethrough.

Here, the colored area indicates an area in which a diagram, a pattern, a figure, color, identification information, and the like are printed by application of pigment.

There is also described herein, an absorbent article, including: a top sheet positioned in a face which is brought into contact with a human body; a back sheet positioned in a face which is opposite to the top sheet and is brought into contact with underwear; and an absorbent body interposed between the top sheet and the back sheet. The back sheet includes a colored area and a non-colored area, and an inspection portion which transmits light outputted from an optical sensor for inspection is provided in the non-colored area.

Here, the non-colored area indicates an area other than the colored area in the back sheet.

Moreover, inspection means discrimination between acceptable and unacceptable products performed with an optical sensor in the inspection process.

Furthermore, the inspection portion is an area of the surface of the back sheet which is irradiated by the light outputted from the optical sensor in the inspection process of the manufacturing process, and the size thereof is arbitrary. Moreover, at least one inspection portion is provided, however, a plurality of inspection portions may be provided.

There is also described herein, an absorbent article, including: a top sheet positioned in a face which is brought into contact with a human body; a back sheet positioned in a face which is opposite to the top sheet and is brought into contact with underwear; and an absorbent body interposed between the top sheet and the back sheet. The back sheet includes a colored area, and an inspection portion in the colored area at which inspection with an optical sensor is performed has a light transmittance allowing light outputted from the optical sensor to be transmitted therethrough.

In the colored area, an identification to identify a front-rear orientation of the absorbent article may be provided.

### Effects of the Invention

According to the present disclosure an absorbent article including a back sheet which includes the colored area but is capable of transmitting the light outputted from the optical sensor is obtained.

Accordingly, the visual effects provided by the back sheet including the colored area provide "healing" and "security" to a woman during menstruation, and the back sheet capable of transmitting the light outputted from the optical sensor enables proper inspection to be performed in the inspection process of the manufacturing process.

According to the present disclosure an absorbent article in which the colored area is provided and the inspection portion transmitting the light outputted from the optical sensor for inspection is provided in the non-colored area is also obtained.

Accordingly, the visual effects provided by the back sheet including the colored area provide "healing" and "security" to a woman during menstruation. Moreover, when a print back sheet is formed by application of pigment to a film, the colored area can be colored using arbitrary pigment which does not transmit the light outputted from the optical sensor and therefore can be more colorful and more varied. Furthermore, the non-colored area with light transmittance allowing the light outputted from the optical sensor to be transmitted enables proper inspection in the inspection process of the manufacturing process.

According to the present disclosure, an absorbent article including the back sheet which includes the inspection portion with light transmittance allowing the light outputted from the optical sensor to be transmitted is also obtained.

Accordingly, not only the visual effects provided by the back sheet including the colored area provide "healing" and "security" to a woman during menstruation, but also the colored area other than the inspection portion can be colored using arbitrary pigment and therefore can be more colorful and more varied. Furthermore, the inspection portion having light transmittance allowing the light outputted from the optical sensor to be transmitted therethrough enables proper inspection in the inspection process.

According to the present disclosure, an absorbent article provided with indication of front and rear on the back sheet is obtained as well.

Accordingly, the front and rear of the absorbent article can be easily determined with the indication of front and rear printed on the back sheet, thus providing better feeling of security and as well as comfortable wearing feeling.

### Brief Description of the Drawings

[FIG. 1] This is a frame format of a sanitary napkin of the first example, to which an absorbent article of the present disclosure is applied.
[FIG. 2] This is a frame format of a rear side of the sanitary napkin of FIG. 1.
[FIG. 3] This is a frame format of a rear side of a sanitary napkin of the second example, to which the absorbent article of the present disclosure is applied.
[FIG. 4] This is a frame format of a rear side of a sanitary napkin of the third example, to which the absorbent article of the present disclosure is applied.
[FIG. 5] This is a frame format of a rear side of a sanitary napkin of the fourth example, to which the absorbent article of the present disclosure is applied.
[FIG. 6] This is a frame format of a rear side of a sanitary napkin of the fifth example, to which the absorbent article of the present disclosure is applied.
[FIG. 7] This is a frame format of a rear side of a sanitary napkin of the sixth example, to which the absorbent article of the present disclosure is applied.
[FIG. 8] This is a frame format of a rear side of a sanitary napkin of the seventh example, to which the absorbent article of the present disclosure is applied.

### Best Mode for Carrying out the Invention

### [First Example]

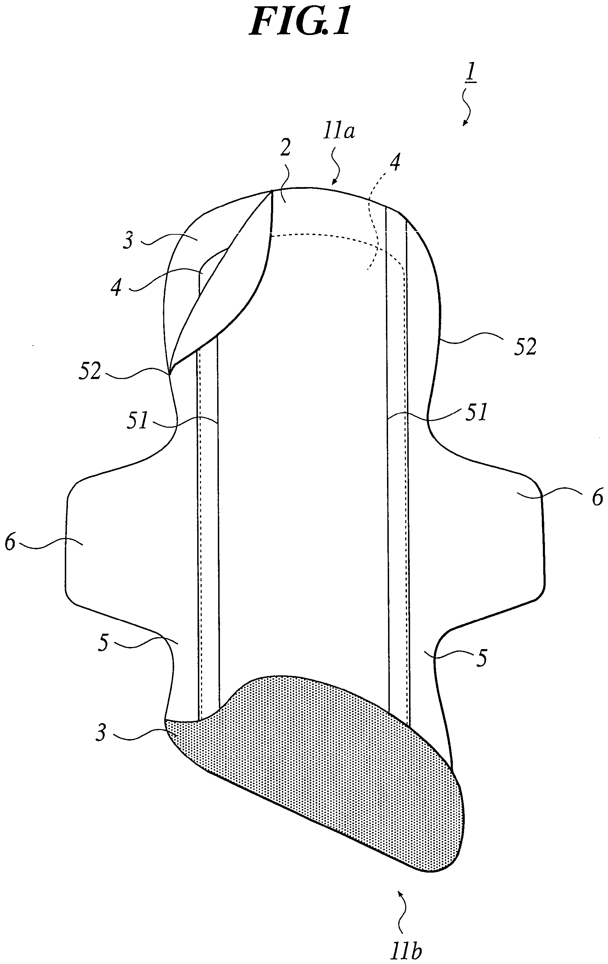

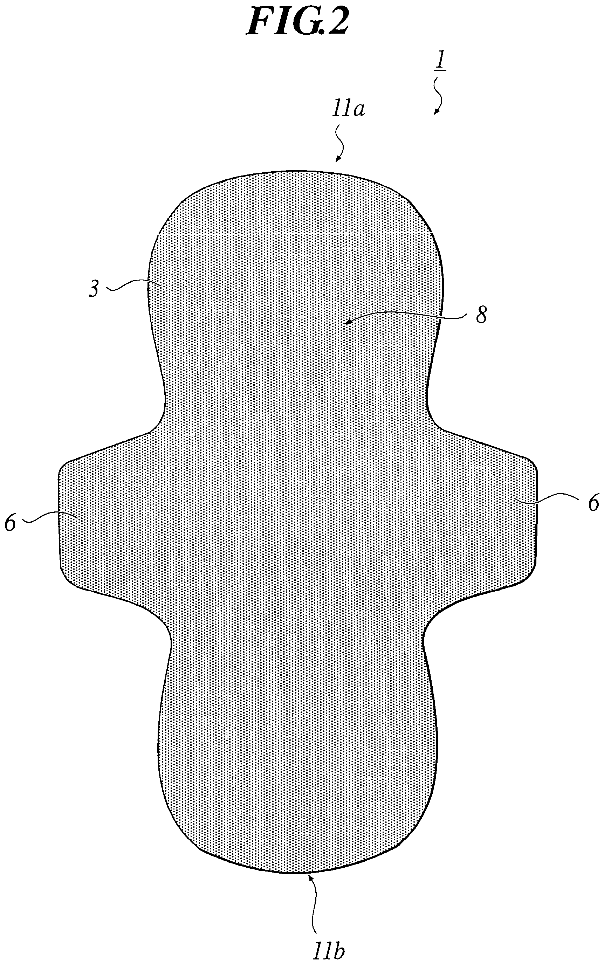

Hereinafter, a description is given for the first example of the present disclosure with reference to FIGS. 1 and 2.

FIG. 1 is a frame format of a sanitary napkin of the first example, to which an absorbent article of the present disclosure is applied.

As shown in FIG. 1, a sanitary napkin (hereinafter, referred to as a "napkin") 1 as an absorbent article includes a top sheet 2 positioned in a face which is brought into contact with a human body, a back sheet 3 positioned in a face which is opposite to the top sheet 2 and is brought into contact with underwear, an absorbent body 4 interposed between the top sheet 2 and back sheet 3, a pair of right and left side sheets 5, 5 provided in edge portions of both sides of the absorbent body 4 in the longitudinal direction from a front end portion 11a to a rear end portion 11b, attachment pieces 6, 6 composed by laterally extending the side sheets 5, 5 and back sheet 3 in the middle portion of the napkin 1 in the longitudinal direction, at both sides, and the like. The sanitary napkin 1 is packaged with a packaging material (not shown).

The top sheet 2 is provided in a skin contact face which is brought into contact with a human body when the napkin 1 is worn on underwear. The top sheet 2 is a liquid-permeable sheet and plays a role of absorbing menstrual blood and transporting the same to the absorbent body 4. Examples of the liquid-permeable sheet are sheets made of porous or non-porous non-woven fabric, porous plastic sheets, and the like. The non-woven fabric is composed of fibers joined or entwined with each other by a chemical method, a mechanical method or a combination thereof without being woven or knitted. Raw material fibers constituting the non-woven fabric can be, in addition to synthetic fibers of olefin type such as polyethylene and polypropylene, polyester type and polyamide type, regenerated fibers such as rayon and cuprammonium rayon, natural fibers such as cotton, and the like. As for the method of manufacturing non-woven fabric, a known method can be arbitrary used. For example, non-woven fabric can be manufactured by the dry method, the wet method, spunlacing, spun bonding, thermal bonding, melt blowing, needle punching, a combination thereof, or the like.

The top sheet 2 includes a plurality of permeable holes. This allows menstrual blood and humor including vaginal discharge to be more quickly absorbed and provide excellent dry touch.

The back sheet 3 is provided in a skin non-contact face opposite to the top sheet 2. The back sheet 3 is a liquid-impermeable and light transmitting sheet and plays a role in preventing the menstrual blood absorbed by the absorbent body 4 from reaching underwear. For the liquid-impermeable sheet, for example, a sheet material with at least water-impermeability, such as polyethylene and polypropylene, is used. In terms of the light transmitting property, 100-percent polyethylene is desirable. In addition, the liquid-impermeable sheet including laminated non-woven fabric laminating non-woven fabric on a polyethylene sheet or the like, a non-woven sheet together with a waterproof film provided to substantially secure the liquid impermeability (in this case, the waterproof film and non-woven fabric constitute the back sheet), and the like can be used. In recent years, moisture permeable materials tend to be suitably used in terms of preventing stuffy feeling. As for this sheet material with the water-impermeability and moisture permeability, for example, a microporous sheet material or the like can be mentioned. The microporous sheet material is obtained by, for example, melting and kneading inorganic filler into olefin resin, such as polyethylene or polypropylene, to form a sheet and then uniaxially or biaxially stretching the same. In this example, an air-permeable polyethylene sheet is used.

Specifically, the back sheet 3 of the first example is made of a colored printing film which is a film of a water-impermeable sheet material such as polyethylene or polypropylene, a liquid-impermeable non-woven sheet, a microporous sheet material, or the like. The back sheet 3 is provided with a colored area 8. As for method to print the film, gravure printing, flexographic printing, ink-jet printing, or the like is used. To obtain the printing film as the back sheet 3, in the present example, C. I. pigment 15 of organic pigment type is used. However, it is also possible to use C. I. pigment blue 27, C. I. pigment yellow 12, C. I. pigment yellow 1, C. I. pigment yellow 14, C. I. pigment yellow 83, C. I. pigment green 7, C. I. pigment violet 19, C. I. pigment violet 23, C. I. pigment orange 13, C. I. pigment red 57-1, C. I. pigment red 57, C. I. pigment red 22, C. I. pigment red 48, C. I. pigment red 166, or the like.

Each of the printing films obtained by using the above pigments has light transmittance of 15% or more. Accordingly, the back sheet 3 including the aforementioned printing films has light transmittance of 15% or more. Here, the light transmittance is set to 15% or more since discrimination inspection by the optical sensor discriminates between acceptable and unacceptable products by a threshold value set to the light transmittance of 15% in a later-described napkin inspection process. The lower limit of the light transmittance of the printing film may be determined depending on this threshold value of the optical sensor.

The light transmittance was measured according to the light transmittance measurement method (JIS K 7105) using a haze• transmittance• reflectance meter HR-100 manufactured by Murakami Color Research Laboratory Co., Ltd.

Here, in case of using pigment which has a light transmittance of less than 15% when formed as the printing film, for example, the amount of the pigment used is set to 0.5 to 20% of the total weight (0.688 g) of the film as the back sheet so that the printing film has a light transmittance of 15% or more.

Furthermore, in case of using pigment which has a light transmittance of less than 15% when formed as the printing film, for example, an area where the pigment is applied is set to 3 to 97% of an area (209.8 cm²) of the back sheet 3 so that the printing film has a light transmittance of 15% or more.

The absorbent body 4 absorbs watery components such as urine as body liquid and the like when the napkin 1 is used, and is composed of an absorbent raw material such as cotton and pulp or an absorbent body core (not shown) formed by a combination of fibers or a sheet-like base material such as a film and highly absorbent resin such as highly absorbent polymer and the like, covered with liquid-permeable crepe paper (not shown). Here, the absorbent body core may have a single layer structure or a multiple layer structure.

Each of the side sheets 5, 5 is formed by a liquid-impermeable non-woven sheet. Each side sheets 5, 5 includes an inner end portion 51, which extends above the top sheet 2, and an outer end portion 52, which is the other end portion and forms an outline of the napkin 1. The side sheet 5 is bonded to the top sheet 2 in the vicinity of the outer end portion 52 so as to be fixed. The side sheet 5 play a role in preventing leakage of body liquid, which is received but cannot be held by the top sheet 2 and absorbent body 4, to the outside. In the present example, non-woven fabric is used for the side sheet 5.

The attachment pieces 6, 6 are, for example, generally called wings or flaps. When the napkin 1 is worn on underwear, the attachment pieces 6, 6 are folded back to the back sheet 3 side, so that a crotch portion of the underwear is sandwiched therebetween and attached to a face of the underwear opposite to a skin contact face, thus playing a role in preventing misalignment of the napkin 1 due to a wearer's movement. The attachment pieces 6, 6 can be formed by extending the top sheet 2 and back sheet 3, but may also be made of separate members. That is, the attachment pieces 6, 6 may be formed using either a liquid-permeable or impermeable material. In the present example, the attachment pieces 6, 6 are formed by extending the side sheet 5 and back sheet 3.

Next, a description is given on the inspection process in the manufacturing process of the napkin 1.

The manufacturing process of the napkin 1 includes the inspection process. In the inspection process, discrimination inspection is performed with an optical sensor to discriminate between acceptable products and so-called unacceptable products, involving issues such as: whether the absorbent body 4 is certainly included between the top sheet 2 and the back sheet 3; in case where it is included, whether the absorbent body 4 is twisted, misaligned or defective; whether a foreign substance is added thereto; and the like. The optical sensor outputs light to the napkin 1, and by the transmittance thereof, or the light transmittance, the napkin 1 is determined to be the acceptable or unacceptable product. Specifically, a napkin with a light transmittance of 15% or more is determined to be an acceptable product, and a napkin with a light transmittance less than 15% is determined to be an unacceptable product.

Accordingly, to properly discriminate between the acceptable and unacceptable products, the back sheet 3 of the napkin 1 needs to have a light transmittance of 15% or more. In other words, it is preferable that the light transmittance of the napkin 1 using the back sheet 3, which is a printing film, is 15% or more. More preferably, the light transmittance of the napkin 1 is 15% to 80% and, further preferably, 15% to 55%. When the light transmittance is more than 80%, a design provided on the back sheet 3 becomes difficult to be observed even when the napkin 1 is not used. When the light transmittance is more than 55%, menstrual blood absorbed by the absorbent body is seen through the back sheet 3 after the napkin 1 is used, and the design of the back sheet 3 becomes difficult to be observed.

With the napkin 1 according to the first example described above, the visual effect provided by the back sheet 3 including the colored area 8 provides "healing" or "security" to a woman during menstruation. In addition, since the napkin 1 is designed to include the back sheet 3 which can transmit the light outputted from the optical sensor, proper inspection can be performed in the inspection process.

### [Second Example]

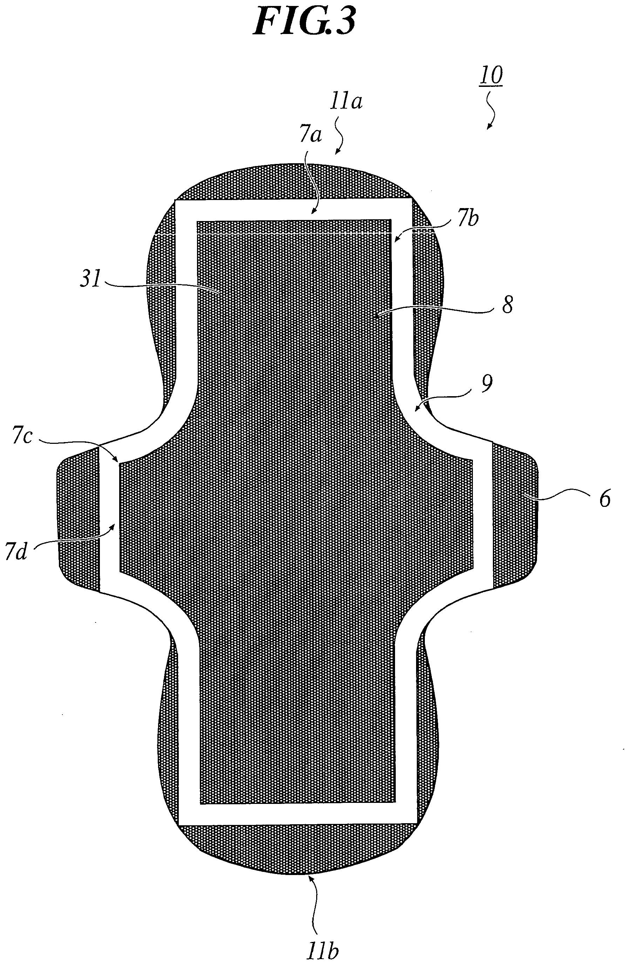

Next, a description is given on the second example of the present disclosure with reference to FIG. 3. The second example is not part of the present invention. FIG. 3 is a frame format of a sanitary napkin of the second example, to which the absorbent article of the present disclosure is applied. Moreover, a napkin 10 in the second example is different from the napkin of the first example in terms of a pattern provided on the back sheet 31. In the description of the second example, components thereof that are same as those of the first example are given the same numerals, and the description thereof is omitted.

As for the back sheet 31 of the napkin 10, a colored printing film is used.

Here, pigment applied to a film to obtain the printing film as the back sheet 31 may be chosen arbitrarily.

In this example, the back sheet 31 includes a colored area 8 and a non-colored area 9. In the non-colored area 9, inspection portions 7a, 7b, 7c, and 7d (hereinafter, referred to as inspection portions 7), at which the discrimination inspection with the optical sensor is performed, are provided.

Here, the inspection portions 7 are regions in a face of the back sheet which are irradiated by the light outputted from the optical sensor in the inspection process of the manufacturing process, and the size thereof is arbitrary. The back sheet 31 should include at least one inspection portion, and may also include a plurality of inspection portions.
In the second example, for example, one inspection portion 7a is provided substantially along a short length direction (width direction) of the napkin 10 in the vicinity of a front end portion 11a of the napkin 10. One inspection portion 7b is provided in an orthogonal direction to the inspection portion 7a, substantially along one of the inner end portions 51 also in the vicinity of the front end portion 11a. Two inspection portions 7c and 7d are provided in base part of the attachment pieces 6. In total, four inspection portions 7 are provided.
In other words, each of the inspection portions 7 is provided in the non-colored area 9. Accordingly, each inspection portion 7 has a light transmittance of 15% or more, and proper judgment of acceptable and unacceptable products can be performed in the inspection process of the manufacturing process.

With the napkin 10 according to the second example described above, the visual effect provided by the back sheet 31 including the colored area 8 provides "healing" or "security" to a woman during menstruation. Moreover, the back sheet 31 allowing proper inspection in the inspection process can be colored with pigment which is applied to a film to form a printing film with a light transmittance of 15% or less. Accordingly, the colored area 8 can be more effective and more varied.

### [Third Example]

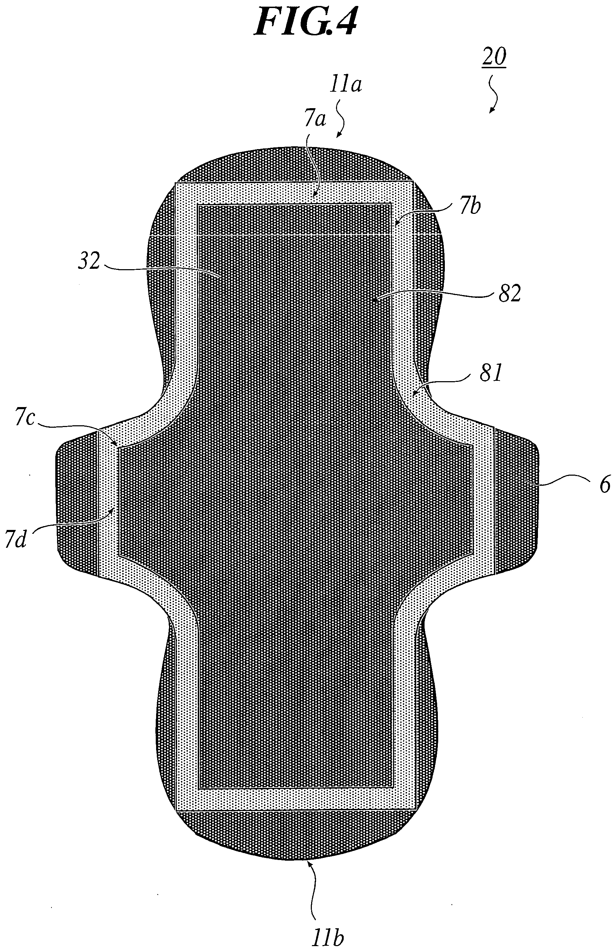

Next, a description is given on the third example of the present disclosure with reference to FIG. 4.

FIG. 4 is a frame format of a sanitary napkin of the third example, to which the absorbent article of the present disclosure is applied.

A napkin 20 in the third example is different from the napkin of the first example in terms of a pattern provided on a back sheet 32. In the description of the third example, components thereof that are same as those of the first example are given the same numerals, and the description thereof is omitted. The inspection portions 7 are provided at the same places as those of the napkin 10 of the second example.

As for the back sheet 32 of the napkin 20, a colored printing film is used.

In this example, the back sheet 32 includes a colored area 8, and the inspection portions are provided in a slightly colored area 81. The slightly colored area 81 where the inspection portions 7 are provided with pigment, which has a light transmittance of 15% or more, when applied to a film to form a printing film. The inspection portions 7 therefore have a light transmittance of 15% or more. Accordingly, even when the light transmittance of most of the back sheet 32 is 15% or less, proper discrimination between acceptable and unacceptable products is reliably performed in the inspection process.

With the napkin 20 according to the third example described above, the visual effect provided by the back sheet 32 including the colored area 8 provides "healing" or "security" to a woman during menstruation. Moreover, the back sheet 32 enabling proper inspection in the inspection process can be colored with pigment which has a light transmittance of 15% or less when applied to a film to form a printing film. The pigment applied to the slightly colored area 81 including the inspection portions 7 is configured to have a color with a tone close to that of pigment applied to a darkly colored area 82, thus preventing the inspection portions 7 from being noticed. The colored area 8 can therefore be a more effective colored area and more excellent in design.

### [Fourth Example]

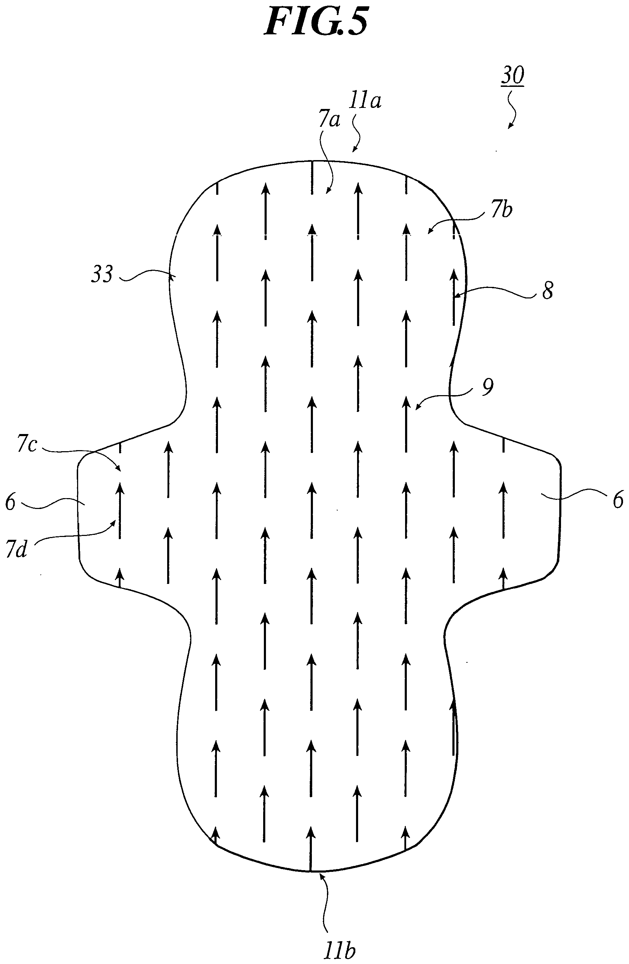

Next, a description is given on the fourth example of the present disclosure with reference to FIG. 5.

FIG. 5 is a frame format of a sanitary napkin of the fourth example, to which the absorbent article of the present disclosure is applied.

A napkin 30 in the fourth example is different from the napkin of the first example in terms of a pattern provided on a back sheet 33. In the description of the fourth example, therefore, components thereof that are same as those of the first example are given the same numerals, and the description thereof is omitted. The inspection portions 7 are provided at the same places as those of the napkin 10 of the second example.

As for the back sheet 33 of the napkin 30, a colored printing film is used.

As shown in FIG. 5, in the back sheet 33, a plurality of arrows are arranged with tips directed in a certain direction, that is, in a direction from 11b toward 11a. The arrows as identifications to identify the front and rear orientation allow a user to easily determine the front and rear of the napkin.

Here, the colored area 8 is each arrow as the identification, and the non-colored area 9 is the other area. To the inspection portions 7, either pigment is not applied, or pigment which has a light transmittance of 15% or more when applied to a film to form a printing film is applied.

With the napkin 3 according to the fourth example described above, reliable inspection is performed in the inspection process of the manufacturing process. Moreover, the front and rear orientation of the napkin 30, which has been difficult to determine when the napkin 30 is being used, can be easily recognized.

### [Fifth Example]

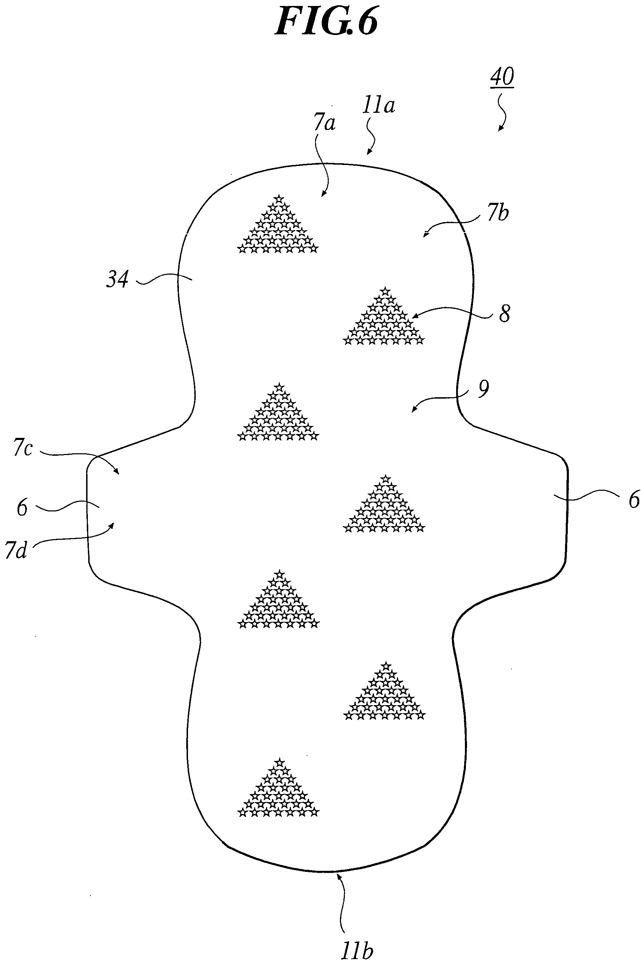

Next, a description is given on the fifth example of the present disclosure with reference to FIG. 6.

FIG. 6 is a frame format of a sanitary napkin of the fifth example, to which the absorbent article of the present disclosure is applied.

A napkin 40 in the fifth example is different from the napkin of the first example in terms of a pattern provided for a back sheet 34. In the description of the fifth example, therefore, components thereof that are same as those of the first example are given the same numerals, and the description thereof is omitted. The inspection portions 7 are provided at the same places as those of the napkin 10 of the second example.

As for the back sheet 34 of the napkin 40, a colored printing film is used.

As shown in FIG. 6, on the back sheet 34, assembly of fine figures that form a substantially triangle are displayed to identify the front and rear orientation, and are arranged with vertices of the triangles directed in a certain direction, that is, in the direction from 11b toward 11a. This arrangement allows a user to easily determine the front and rear thereof.

At this time, the colored area 8 is each of the assembly of fine figures that form a substantially triangle, and the non-colored area 9 is an area other than the substantially triangles. To the inspection portions 7, either pigment is not applied, or pigment which has a light transmittance of 15% or more when applied to a film to form a printing film is applied.

### [Sixth Example]

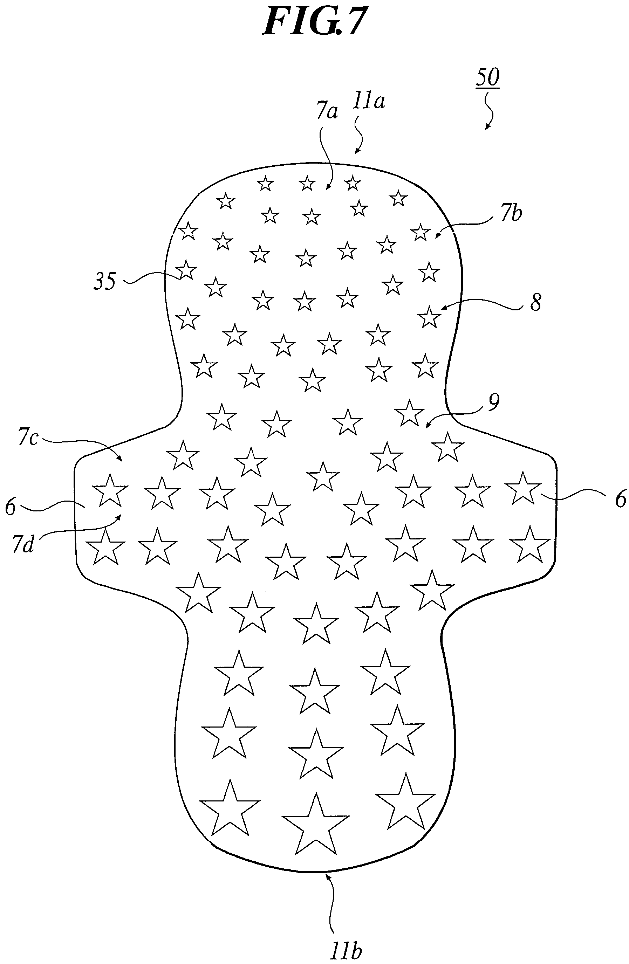

Next, a description is given on the sixth example of the present disclosure with reference to FIG. 7.

FIG. 7 is a frame format of a sanitary napkin of the sixth example, to which the absorbent article of the present disclosure is applied.

A napkin 50 in the sixth example is different from the napkin of the first example in terms of a pattern provided on a back sheet 35. In the description of the sixth example, therefore, components thereof that are same as those of the first example are given the same numerals, and the description thereof is omitted. The inspection portions 7 are provided at the same positions as those of the napkin 10 of the second example.

As for the back sheet 35 of the napkin 50, a colored printing film is used.

As shown in FIG. 7, on the back sheet 35, a pattern with a plurality of stars is provided across the entire surface. The pattern is designed so that stars drawn in the vicinity of 11b have lager areas than the stars drawn in the vicinity of 11a, and the size of the stars is gradually reduced from 11b toward 11a. With the pattern in which the area of the stars as the identifications to identify the front and rear orientation changing toward a certain direction, the user can easily determine the front and rear.
At this time, the colored area 8 is the pattern of the stars, and the non-colored area 9 is an area other than the pattern of the stars. To the inspection portions 7, either pigment is not applied, or pigment which has a light transmittance of 15% or more when applied to a film to form a printing film is applied.

### [Seventh Example]

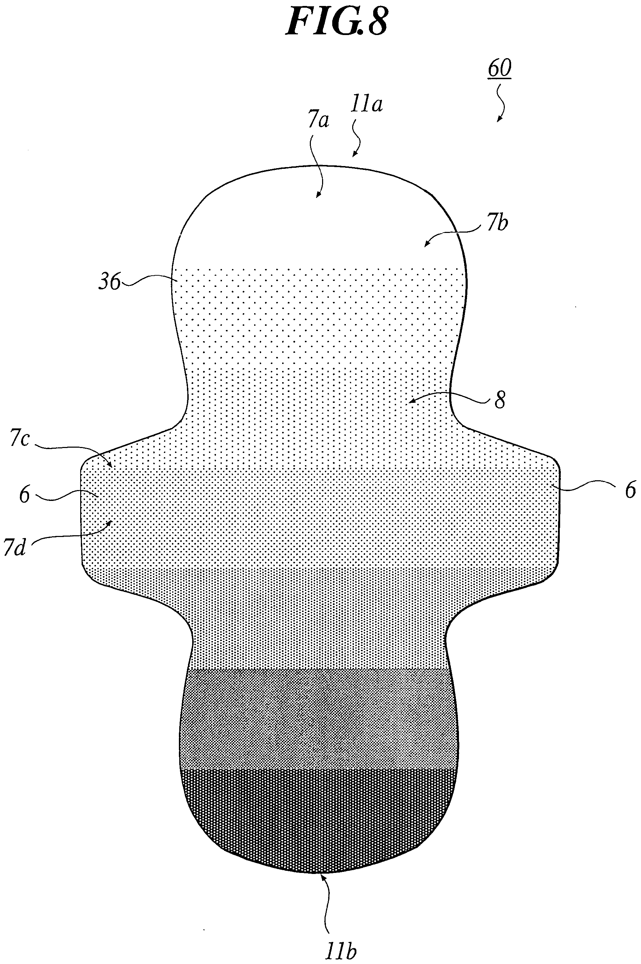

Next, a description is given on the seventh example of the present disclosure with reference to FIG. 8.

FIG. 8 is a frame format of a sanitary napkin of the seventh example, to which the absorbent article of the present disclosure is applied.

A napkin 60 in the seventh example is different from the napkin of the first example in terms of a pattern provided for a back sheet 36. In the description of the seventh example, therefore, the components thereof that are same as those of the first example are given the same numerals, and the description thereof is omitted. The inspection portions 7 are provided at the same positions as those of the napkin 10 of the second example.

As for the back sheet 36 of the napkin 60, a colored printing film is used.

As shown in FIG. 8, the back sheet 36 has a gradation of color from dark to light from 11b to 11a. With the pattern in which color density as the identification to identify the front and rear orientation changing toward a certain direction, the user can easily determine the front and rear.

At this time, the colored area 8 is the entire surface of the back sheet 3. To the inspection portions 7, either pigment is not applied, or pigment which has a light transmittance of 15% or more when applied to a film to form a printing film is applied. It is noted that in examples 2-7, these are only part of the present invention in the cases where pigment is applied to the inspection portions 7.

As for the method of forming the designs relating to the above described examples, for example, heat embossing or embossing can be used. The heat embossing and embossing are effective especially in the examples in which the pattern as the colored area is provided in the area of the back sheet that is not colored, using a pigment (Examples of fifth through seventh).

The heat embossing is a method in which a film of a thermoplastic resin sheet is heat compressed and the resin is partially melted at the heated part for bonding to form a protrusion at each embossed portion. Using this method can additionally provide an embossed pattern on the printing film in which pigment is already applied to form the colored area.

The embossing is a processing method in which a film is compressed to form a protrusion at each embossed portion in the film by the compressing force. This method can also additionally provide an embossed pattern on the colored printing film.

With heat embossing and embossing, on the printing film including the colored area provided by application of pigment, pattern can be formed without using pigment. Accordingly, the light transmittance of the printing film is not reduced, and the discrimination inspection can be reliably performed in the inspection process.

When pigment is applied to a film to form the printing film, especially when a large amount of pigment is used in order to be applied all over the film, it takes much time to dry the pigment. If the manufacturing process is progressed to the subsequent process before the pigment is fixed to the film, not only the pigment adheres to line or facilities, but also a preferable printed state cannot be maintained. However, on the other hand, drying the pigment sufficiently takes much time, and the manufacturing efficiency is reduced. Accordingly, pigment is applied to the film, and a hot melt adhesive is additionally applied to the film. The pigment is therefore covered with the hot melt adhesive, thus making it possible to not only maintain the preferable printed state but also prevent the reduction in the efficiency.

Furthermore, for example, using water-soluble ink as the pigment to form the printing film allows the status of usage to be easily determined, since the pattern provided for the back sheet disappears when the napkin 1 absorbs moisture.

Moreover, the pattern provided for the back sheet is not limited to the pattern allowing the front and rear to be identified and may be an entirely uniform pattern such as stripe or check pattern.

### Industrial Applicability

The present disclosure is applicable to manufacturing absorbent articles.

### Description of Reference Numerals

- 1: SANITARY NAPKIN
- 2: TOP SHEET
- 3: BACK SHEET
- 4: ABSORBENT BODY
- 5: SIDE SHEET
- 6: ATTACHMENT PIECE
- 7: INSPECTION PORTION
- 8: COLORED AREA
- 9: NON-COLORED AREA
- 10: SANITARY NAPKIN
- 11A: FRONT END PORTION
- 11B: REAR END PORTION
- 20: SANITARY NAPKIN
- 30: SANITARY NAPKIN
- 31: BACK SHEET
- 32: BACK SHEET
- 33: BACK SHEET
- 34: BACK SHEET
- 35: BACK SHEET
- 36: BACK SHEET
- 40: SANITARY NAPKIN
- 50: SANITARY NAPKIN
- 51: INNER END PORTION
- 52: OUTER END PORTION
- 60: SANITARY NAPKIN
- 81: LIGHT COLORED AREA
- 82: DARK COLORED AREA

## Claims

1. An absorbent article (1), comprising:
a top sheet (2) positioned in a face which is brought into contact with a human body;
a back sheet (3) positioned in a face which is opposite to the top sheet (2) and is brought into contact with underwear; and
an absorbent body (4) interposed between the top sheet (2) and the back sheet (3), wherein
the back sheet (3) includes a coloured area (8) printed by application of a pigment to a film, and an inspection portion (7) in the coloured area at which inspection with an optical sensor is performed has a light transmittance of 15% or more allowing light outputted from the optical sensor to be transmitted therethrough.

2. The absorbent article (1) of claim 1, wherein the coloured pigment is configured such that the back sheet (3) has a transmittance of 15-55% in the area (8) of coloured pigment.

3. The absorbent article (20) of claim 1 or 2, wherein the coloured area (8) comprises first (81) and second (82) areas of coloured pigment, the inspection portion (7) provided in the first area (81), and wherein the coloured pigment in the second area (82) has a light transmittance of 15% or less.

4. The absorbent article (20) of claim 3, wherein the coloured pigments in the first (81) and second (82) areas are configured to have colours of similar tone.

5. The absorbent article (30, 40, 50, 60) of any preceding claim, wherein in the coloured area, an identification to identify a front-rear orientation of the absorbent article (30, 40, 50, 60) is provided.

6. The absorbent article (30, 40, 50, 60) of any preceding claim, wherein the light transmittance is measured according to the light transmittance measurement method JIS K 7105 using a haze transmittance reflectance meter HR-100.

## Patentansprüche

1. Absorbierender Gegenstand (1), der umfasst:
eine obere Bahn (2), die auf einer Fläche positioniert ist, die mit einem menschlichen Körper in Berührung gebracht wird;
eine hintere Bahn (3), die auf einer Fläche positioniert ist, die der oberen Bahn (2) gegenüberliegt und mit Unterwäsche in Berührung gebracht wird; und
einen absorbierenden Körper (4), der zwischen der oberen Bahn (2) und der hinteren Bahn (3) eingefügt ist, wobei
die hintere Bahn (3) einen Farbbereich (8), der durch Auftragung eines Pigments auf eine Folie bedruckt wird, und einen Prüfungsabschnitt (7) in dem Farbbereich beinhaltet, in dem eine Prüfung mit einem optischen Sensor durchgeführt wird, der eine Lichtdurchlässigkeit von wenigstens 15 % aufweist, die es dem von dem optischen Sensor ausgegebenem Licht ermöglicht, dahindurch durchgelassen zu werden.

2. Absorbierender Gegenstand (1) nach Anspruch 1, wobei das Farbpigment derart konfiguriert ist, dass die hintere Bahn (3) in dem Bereich (8) des Farbpigments eine Durchlässigkeit von 15-55 % aufweist.

3. Absorbierender Gegenstand (20) nach Anspruch 1 oder 2, wobei der Farbbereich (8) einen ersten (81) und einen zweiten (82) Bereich des Farbpigments umfasst, wobei der Prüfungsabschnitt (7) in dem ersten Bereich (81) bereitgestellt ist und wobei das Farbpigment in dem zweiten Bereich (82) eine Lichtdurchlässigkeit von höchstens 15 % aufweist.

4. Absorbierender Gegenstand (20) nach Anspruch 3, wobei die Farbpigmente in dem ersten (81) und dem zweiten (82) Bereich konfiguriert sind, um Farben eines ähnlichen Farbtons aufzuweisen.

5. Absorbierender Gegenstand (30, 40, 50, 60) nach einem der vorhergehenden Ansprüche, wobei in dem Farbbereich eine Identifikation bereitgestellt ist, um eine Vorderseite/Rückseite-Ausrichtung des absorbierenden Gegenstands (30, 40, 50, 60) zu identifizieren.

6. Absorbierender Gegenstand (30, 40, 50, 60) nach einem der vorhergehenden Ansprüche, wobei die Lichtdurchlässigkeit gemäß dem Lichtdurchlässigkeitsmessverfahren JIS K 7105 unter Verwendung eines Trübungsdurchlässigkeits-Reflexionsgradmessers HR-100 gemessen wird.

## Revendications

1. Article absorbant (1), comprenant :
une feuille supérieure (2) positionnée dans une face qui est mise en contact avec un corps humain ;
une feuille arrière (3) positionnée dans une face opposée à la feuille supérieure (2) et mise en contact avec des sous-vêtements; et
un corps absorbant (4) interposé entre la feuille supérieure (2) et la feuille arrière (3),
la feuille arrière (3) comportant une zone colorée (8) imprimée par application d'un pigment sur un film, et une partie d'inspection (7) dans la zone colorée à laquelle l'inspection avec un capteur optique est effectuée, a une transmittance de lumière de 15 % ou plus permettant à la lumière émise par le capteur optique d'être transmise à travers celui-ci.

2. Article absorbant (1) selon la revendication 1, le pigment coloré étant conçu de telle sorte que la feuille arrière (3) a une transmittance de 15 à 55 % dans la zone (8) du pigment coloré.

3. Article absorbant (20) selon la revendication 1 ou 2, la zone colorée (8) comprenant des première (81) et seconde (82) zones de pigment coloré, la partie d'inspection (7) fournie dans la première zone (81), et le pigment coloré dans la seconde zone (82) ayant une transmittance de lumière de 15 % ou moins.

4. Article absorbant (20) selon la revendication 3, les pigments colorés dans les première (81) et seconde (82) zones étant conçus pour avoir des couleurs de ton similaires.

5. Article absorbant (30, 40, 50, 60) selon l'une quelconque des revendications précédentes, dans la zone colorée, une identification pour identifier une orientation avant-arrière de l'article absorbant (30, 40, 50, 60) étant fournie.

6. Article absorbant (30, 40, 50, 60) selon l'une quelconque des revendications précédentes, la transmittance de lumière étant mesurée conformément au procédé de mesure de la transmittance de lumière JIS K 7105 à l'aide d'un appareil de mesure de réflectance de la transmittance de diffusion globale HR-100.
